# EUROPEAN PATENT SPECIFICATION

(11) **EP 2 063 819 B1**
(45) Date of publication and mention of the grant of the patent: **19.05.2021**
(21) Application number: 07814898.8
(22) Date of filing: 18.09.2007
(51) Int. Cl.: A61F 2/44

(54) **PIVOTABLE VERTEBRAL SPACER**
SCHWENKBARES WIRBELABSTANDSELEMENT
ESPACEUR VERTÉBRAL PIVOTANT

(30) Priority: 18.09.2006 US 845488 P; 17.09.2007 US 856483
(43) Date of publication of application: 03.06.2009
(73) Proprietor: Beacon Biomedical, LLC, Tequesta, Florida 33469 (US)
(72) Inventor: WARNICK, David R., Spanish Fork, UT 84660 (US); ENSIGN, Michael D., Salt Lake City, UT 84124 (US)
(74) Representative: Inspicos P/S
(86) International application number: PCT/US2007/078737
(87) International publication number: WO 2008/036636

(56) References cited:
- FR-A1- 2 858 210
- US-A- 6 159 215
- US-A1- 2005 033 437
- US-A1- 2005 096 745
- US-B1- 6 319 257
- US-B2- 6 599 294

## Description

### FIELD

The present system and method relate to bone fixation devices. More particularly, the present system relates to a spinal implant for interbody fusion to the spinal column.

### BACKGROUND

The degeneration of the intervertebral disk, in particular, the degeneration of the nucleus pulposus, results in a loss of height in the affected disk space which is associated with a weakening of the annulus fibrosus and of the ligaments. As a consequence the spinal column becomes instable and is more susceptible to horizontal displacement of the vertebral bodies with respect to one another. This horizontal movement of vertebral bodies results in impairments of the nerve roots in this region and/or of the spinal marrow with pain resulting therefrom.

The principle treatment of these symptoms consists of the surgical removal of the nucleus pulposus and the insertion of support bodies in order to restore the normal height of the disk space.

There are a variety of demands on both the surgeon performing an intervertebral disk procedure and on the spinal spacers themselves.

A Transforaminal Lumbar Interbody Fusion (TLIF) is a surgical procedure that uses a posterior and lateral approach to access the disc space. To gain access to the disc space, typically a facet joint is removed and access is gained via the nerve foramen. While more technically demanding of the surgeon than other fusion techniques, a TLIF offers a number of clinical advantages. Specifically, when compared to a PosteroLateral Fusion (PLF), a TLIF approach leaves much more of the soft tissue intact, which is less traumatic for the patient. Further, a PLF does not provide access to the disc space.

While a PosteroLateral InterBody Fusion (PLIF) provides access to the disc space, a TLIF approach also provides access to the interbody space, but without the need for manipulation of neural elements, reducing the risk of post-operative neural deficit. Additionally, in a TLIF, only a single spacer is placed. More specifically the TLIF spacer is placed in the anterior aspect of the disc space, thus providing space for a substantial fusion mass in the posterior aspect of the disc space where the natural compression occurs.

However, traditional TLIF procedures do suffer from shortcomings. For example, to place the desired spacer in the anterior aspect of the disc space from an oblique posterior approach, traditional procedures demand that the spacer be released from the inserter and then tamped into place. The two step insertion of this spacer is generally recognized among surgeons as cumbersome FR 2 858 210 A1 discloses an interbody spacer system comprising an interbody spacer and an insertion instrument, the interbody spacer and the instrument having frictionally engaging features, wherein the interbody spacer can be in a first position, a second position which allows the spacer to rotate and a third position providing restrain of the spacer by locking its rotation.

### SUMMARY

The interbody spacer system of the invention is defined in the appended claims. According to one example, a pivotable interbody spacer includes a body defining an inner cavity and a plurality of teeth formed on one end of said spacer.

According to another example, a pivotable interbody spacer system includes an spacer including a body defining an inner cavity and a plurality of teeth formed on one end of the spacer, and an insertion instrument associated with the spacer, wherein the insertion instrument includes a retractable latching mechanism and matching interior teeth configured to pivotably mate with said teeth formed on said spacer.

Furthermore, according to one example, a method for pivotably implanting an interbody spacer includes coupling the spacer to a pivoting instrument, inserting the spacer through a nerve foramen, simultaneously pivoting and inserting the spacer until the spacer is inserted to its final position, and releasing the spacer from the instrument to facilitate removal of the instrument.

### BRIEF DESCRIPTION OF THE DRAWINGS

The accompanying drawings illustrate various exemplary embodiments of the present system and method and are a part of the specification. Together with the following description, the drawings demonstrate and explain the principles of the present system and method. The illustrated embodiments are examples of the present system and method and do not limit the scope thereof.
**FIGS. 1A and 1B** are a side view and a perspective view, respectively, of a pivotable interbody spacer, according to one exemplary embodiment.
**FIGS. 2A and 2B** are perspective views of an insertion instrument configured to grasp and manipulate the pivotable interbody spacer of FIGS. 1A and 1B, according to one exemplary embodiment.
**FIGS. 3A and 3B** are side views of a pivotable interbody spacer and an insertion instrument, according to one exemplary embodiment.
**FIGS. 4A and 4B** are side views of an insertion instrument rotatably grasping a pivotable interbody spacer, according to one exemplary embodiment.
**FIGS. 5A and 5B** are side views of an insertion instrument fixably grasping a pivotable interbody spacer, according to one exemplary embodiment.
**FIG. 6** is a flow chart illustrating a method of pivotably inserting an interbody spacer, according to one exemplary embodiment.
**FIG. 7** is a side view of the pivotable interbody spacer being inserted using the method of FIG. 6, according to various exemplary embodiments.
**FIG. 8** is a side view of pivotably inserting an interbody spacer using the method of FIG. 6, according to various exemplary embodiments.
**FIGS. 9A and 9B** are side views of pivotably inserting an interbody spacer using the method of FIG. 6, according to various exemplary embodiments.
**FIG. 10** is a side view of an interbody spacer in place and the insertion instrument being disengaged and withdrawn, according to various exemplary embodiments.

In the drawings, identical reference numbers identify similar elements or acts. The sizes and relative positions of elements in the drawings are not necessarily drawn to scale. For example, the shapes of various elements and angles are not drawn to scale, and some of these elements are arbitrarily enlarged and positioned to improve drawing legibility. Further, the particular shapes of the elements as drawn, are not intended to convey any information regarding the actual shape of the particular elements, and have been solely selected for ease of recognition in the drawings. Throughout the drawings, identical reference numbers designate similar but not necessarily identical elements.

### DETAILED DESCRIPTION

The present specification describes a system and a method for pivotably inserting an interbody spacer, such as during a Transforaminal Lumbar Interbody Fusion (TLIF). According to one exemplary embodiment, a system including a pivotable interbody spacer and insertion instrument configured to pivotably manipulate the interbody spacer are provided herein. According to one exemplary embodiment, a plurality of teeth is formed on at least one end of the spacer and matching teeth are formed on an insertion instrument. According to one exemplary embodiment, the insertion instrument includes two opposing jaws configured to couple to an end of the pivotable interbody spacer. Further details of the present exemplary system and method will be provided below, with reference to the figures.

In the following description, certain specific details are set forth in order to provide a thorough understanding of various embodiments of the present system and a method for pivotably inserting an interbody spacer. However, one skilled in the relevant art will recognize that the present exemplary system and method may be practiced without one or more of these specific details, or with other methods, components, materials, etc. In other instances, well-known structures associated with interbody fusion have not been shown or described in detail to avoid unnecessarily obscuring descriptions of the present exemplary embodiments.

Unless the context requires otherwise, throughout the specification and claims which follow, the word "comprise" and variations thereof, such as, "comprises" and "comprising" are to be construed in an open, inclusive sense, that is as "including, but not limited to."

Reference in the specification to "one embodiment" or "an embodiment" means that a particular feature, structure, or characteristic described in connection with the embodiment is included in at least one embodiment. The appearance of the phrase "in one embodiment" in various places in the specification are not necessarily all referring to the same embodiment. Furthermore, the particular features, structures, or characteristics may be combined in any suitable manner in one or more embodiments.

### Exemplary Structure

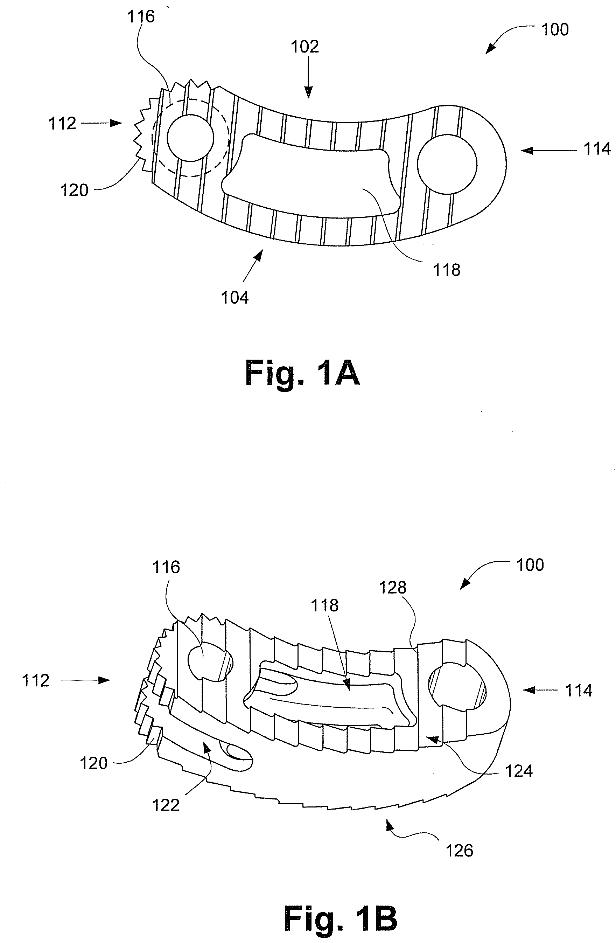

FIGS. 1A and 1B illustrate an interbody spacer (100), according to one exemplary embodiment. As illustrated in FIGS. 1A and 1B, the present exemplary interbody spacer (100) is designed for use as an intervertebral spacer in spinal fusion surgery, where portions of an affected disc are removed from between two adjacent vertebrae and replaced with an spacer that provides segmental stability and allows for bone to grow between the two vertebrae to bridge the gap created by disk removal.

As shown, the present exemplary interbody spacer (100) has an arcuate, "rocker-like" shape with concave anterior face (102) and a convex posterior face (104) to facilitate the insertion of the spacer through a narrow approach window into the disk space. As illustrated, the present interbody spacer (100) includes a proximal end (112) that will be closest to a surgeon during use, and a distal end (114) that will likely be the leading edge of insertion during use.

The central portion of the spacer body may have a variety of apertures and bores designed to facilitate and support bone growth. In one exemplary embodiment, the spacer (100) has a substantially hollow center (118). With the advent of bone morphogenic protein (BMP), the spacer is now seen as a potential delivery tool of the BMP. Consequently, many spacers are now hollow. However, the size of the internal cavity of the spacer is limited by the need to limit the size of the spacer and to maximize the surface area of the spacer. Too large of a spacer will not provide space for a fusion mass. Too small of a surface area will lead to subsidence of the spacer into the adjacent vertebral bodies. According to one embodiment, the spacer is hollowed out to increase cavity volume and surface area while minimizing overall size. Consequently, the present exemplary interbody spacer (100) employs geometry that provides for a small spacer with relatively large surface area and internal cavity (118). Other cavities and geometries may be included in the spacer structure, such as a hollow transverse cylinder (116).

According to one exemplary embodiment, the interbody spacer (100) has an upper face (124) and an opposing lower face (126). A series of ridges (128) traverse the upper and lower faces (124, 126). The ridges (128) are configured to facilitate the insertion of the interbody spacer (100) by preventing retrograde motion and slippage during the insertion process. After the surgery is complete, the ridges (128) also may provide increase surface area, encourage bone growth, and/or prevent dislocation of the interbody spacer (100).

Additionally, as illustrated in FIGS. 1A and 1B, the present interbody spacer (100) includes a plurality of teeth (120) or other frictionally engaging features on at least one end of the spacer (100). According to one exemplary embodiment, any number of protruding features, materials, or rough surface finishes disposed on at least one end of an interbody spacer (100) may be used to pivotably direct an interbody spacer (100) during surgical insertion. Particularly, according to one exemplary embodiment, the interbody spacer (100) has a plurality of teeth (120) disposed about the perimeter of the rounded proximal end (112). The teeth (120) allow for an insertion instrument to firmly grip the interbody spacer (100) and aid in the manipulation of the interbody spacer (100) during insertion. In one exemplary embodiment, the teeth (120), when coupled with an appropriate insertion instrument, may simplify a TLIF procedure by allowing the pivoting and tamping of the interbody spacer (100) without having to release it from the grasp of the insertion instrument.

As shown more particularly in FIG. 1B, the proximal end (112) of the interbody spacer (100) has an access gap (122) that allows access to internal features of the interbody spacer (100), such as the outer surface of the transverse cylinder (116) illustrated in FIGS. 1A and 1B. In alternative embodiments, the teeth (120) or other frictionally engaging features may be formed inside the access gap (122). For ease of explanation only, the present specification will describe the present system and method in the context of an spacer including external teeth (120) on at least one end thereof. In the illustrated embodiment, the distal end (114) of the interbody spacer (100) has a double elliptical leading edge for ease of insertion through the overlying tissues and into the intervertebral space.

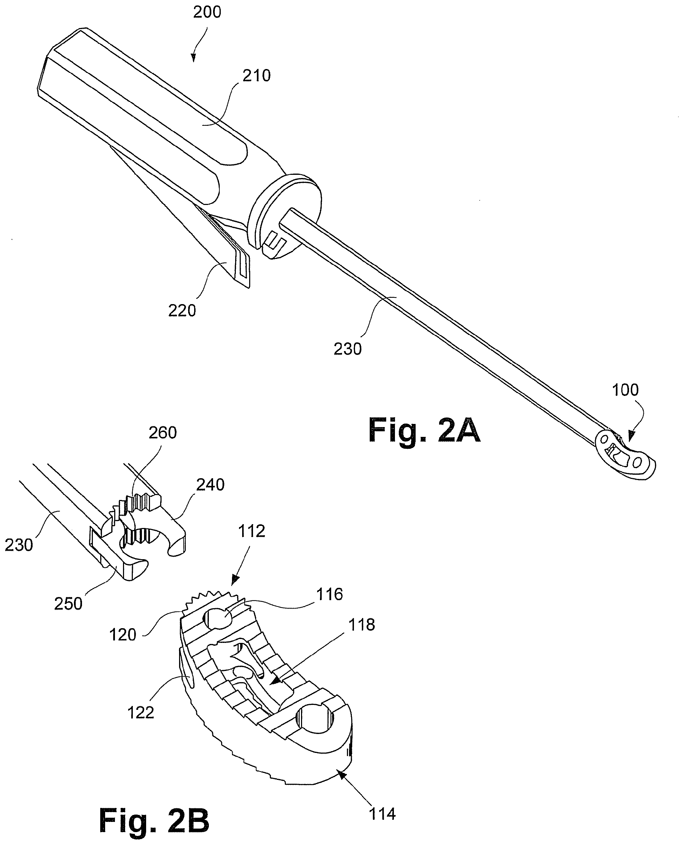

FIG. 2A and 2B illustrate one exemplary insertion instrument (200) that may be used with the present pivotable interbody spacer (100). As illustrated in FIG. 2A, the insertion instrument (200) includes a handle (210) configured to facilitate manual grasping of the insertion instrument (200), a lever (220) pivotably connected to the handle (210), and a shaft (230) extending from one end of the handle (210). At a distal end of the shaft (230) a number of features are disposed which facilitate the grasping and subsequent manipulation of the pivotable interbody spacer (100), according to one exemplary embodiment.

As shown in FIG. 2B, the shaft (230) terminates in a number of features configured to facilitate the grasping and subsequent manipulation of the interbody spacer (100) including opposing jaws (240, 250) and frictional features designed to engage with the frictional features on the interbody spacer (100). In one exemplary embodiment the jaws can pass into the access gap (122) and around the outer perimeter of the transverse cylinder (116) to couple the interbody spacer (100) to the insertion tool (200). Additionally, the distal end of the shaft (230) has a concave surface with a plurality of teeth (260) that is configured to receive and engage the plurality of spacer teeth (120) disposed about the perimeter of the convex surface of the proximal end (112) of the interbody spacer (100).

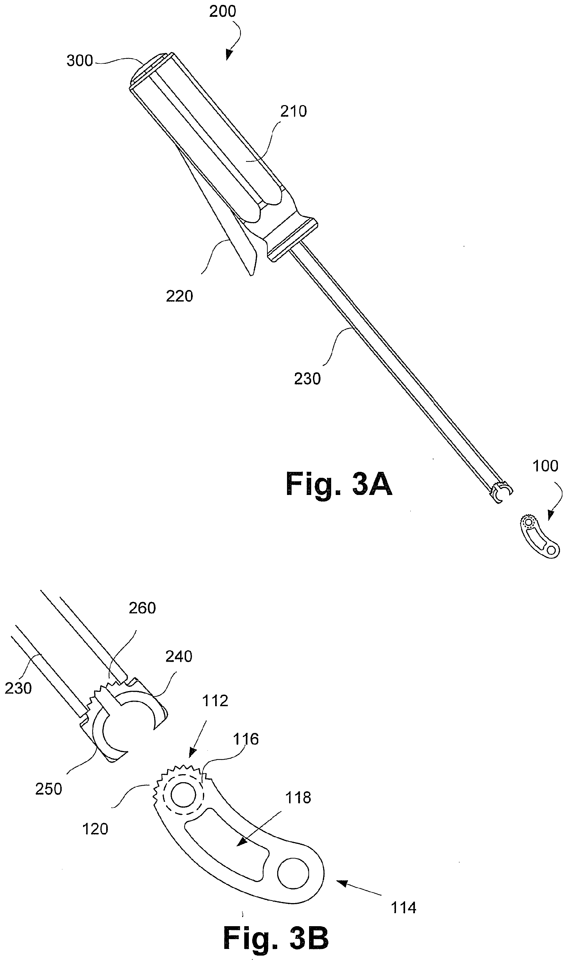

FIGS. 3A and 3B illustrate a side view of one exemplary embodiment of an interbody spacer (100) and an insertion instrument (200). The lever (220) is mechanically connected through interior mechanisms (not shown) to the opposing jaws (240, 250) at the terminal end of the shaft (230). The lever (220) can be moved into three positions that correspond to three different jaw configurations. In FIG. 3A, the lever (220) is shown in a first position that corresponds to an open jaw configuration. As shown in FIG. 3B, in the open jaw configuration, the first jaw (240) and the second jaw (250) are extended out of the shaft body and apart from each other. In this open configuration, the jaws can pass into the access gap (122) and around the outer perimeter of the transverse cylinder (116). The spacing between the jaws (240, 250) can be such that the jaws (240,250) pass around the perimeter of the transverse cylinder (116) without substantial resistance, or such that the spacer "snaps" into the internal cylindrical space created between the jaws (240, 250).

Also shown in FIG. 3A is an impaction cap (300) on the end of the handle (210). The impaction cap (300) provides a durable and resilient surface for impacting the insertion instrument (200) to tamp the interbody spacer (100) into position.

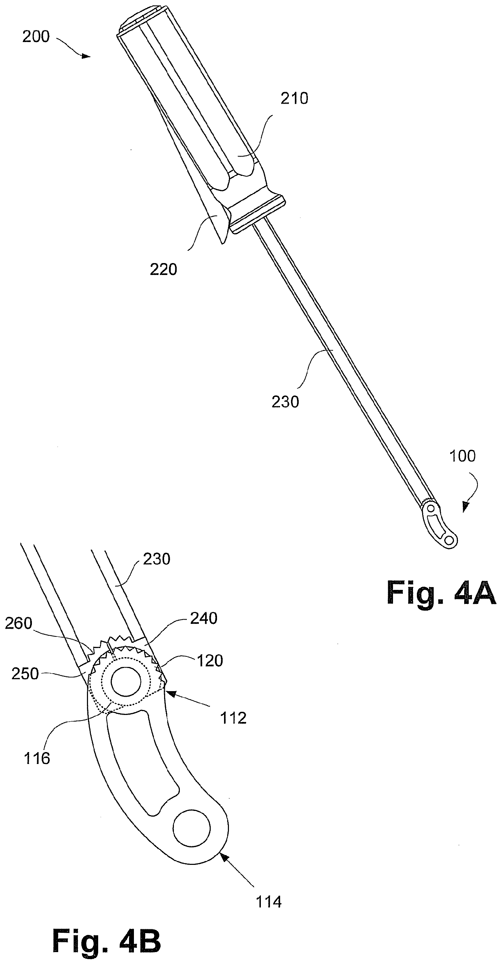

FIGS. 4A and 4B illustrate side views of the insertion instrument (200) and the interbody spacer (100). FIG. 4A shows the lever (220) in a second position which corresponds to a closed jaw configuration. In the closed jaw configuration, the opposing jaws (240, 250) are brought together around the outer perimeter of the transverse cylinder (116). The interbody spacer (100) is firmly grasped by the jaws (240, 250) in a manner that still allows the outer perimeter of the transverse cylinder (116) to rotate within the internal space created by the closed jaws (240, 250) without allowing the spacer (100) to be disengaged from the insertion instrument (200).

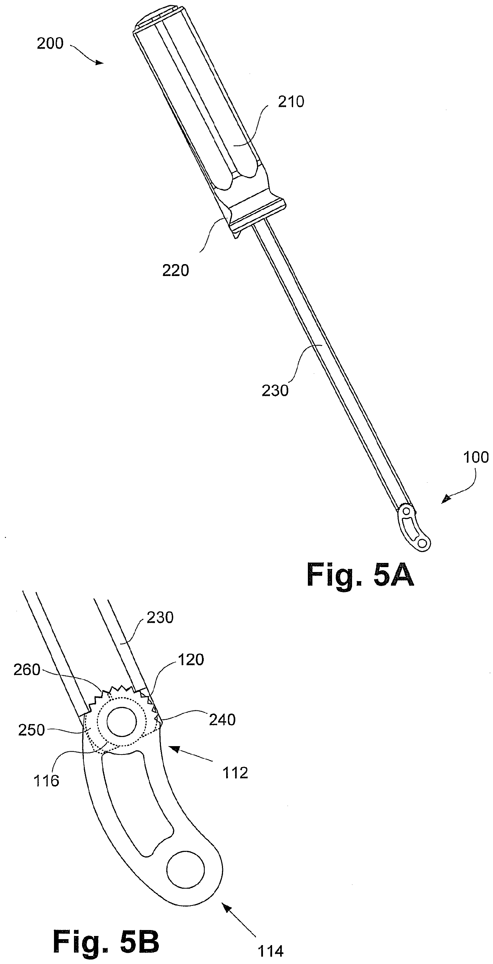

FIGS. 5A and 5B illustrate side views of the insertion instrument (200) and the interbody spacer (100). FIG. 5A shows the lever (220) in a third position which further restrains the spacer (100) by locking its rotation. This is particularly useful during impaction of the spacer (100) into position in the intervertebral space. In one exemplary embodiment the third lever position corresponds to a closed and retracted jaw configuration. In the closed and retracted jaw configuration the opposing jaws (240, 250) are brought together around the outer perimeter of the transverse cylinder (116) and the jaws (240, 250) are retracted into the shaft (230) such that the frictional features on the shaft (260) engage the corresponding frictional features (120) on the spacer. The engagement of the frictional features prevents the pivoting of the spacer (100) with the jaws (240, 250). For example, while the lever (220, FIG. 4A) is the second position, the spacer (100) can be pivoted into the desired angular position with respect to the insertion instrument (200). Then with the spacer (100) at the desired angle, the lever (220) is moved into a third position which retracts the jaws into the shaft, thereby engaging the shaft teeth (260) with spacer teeth (120). The interbody spacer (100) is firmly grasped by the jaws (240, 250) and locked at the desired angle by the engagement of the mating teeth (120, 260). The spacer (100) can now be impacted into the disc space. The position and motion of the rigidly held spacer (100) can be precisely controlled by manipulating the insertion instrument (200).

### Exemplary Method

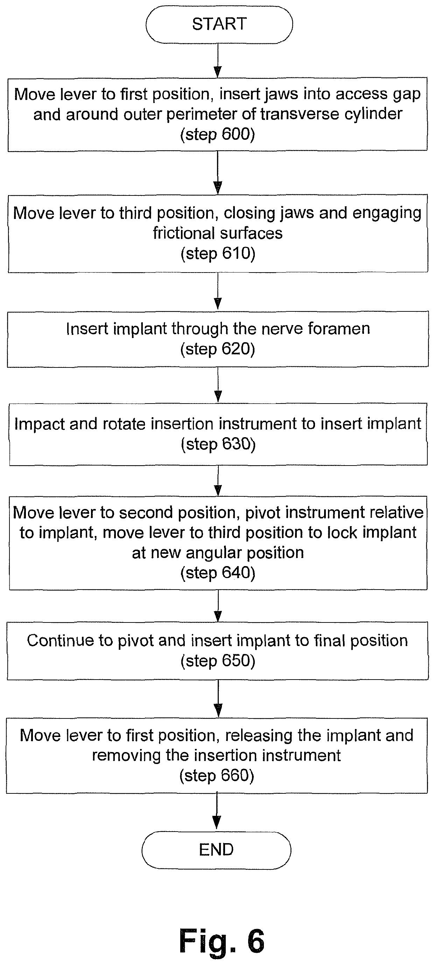

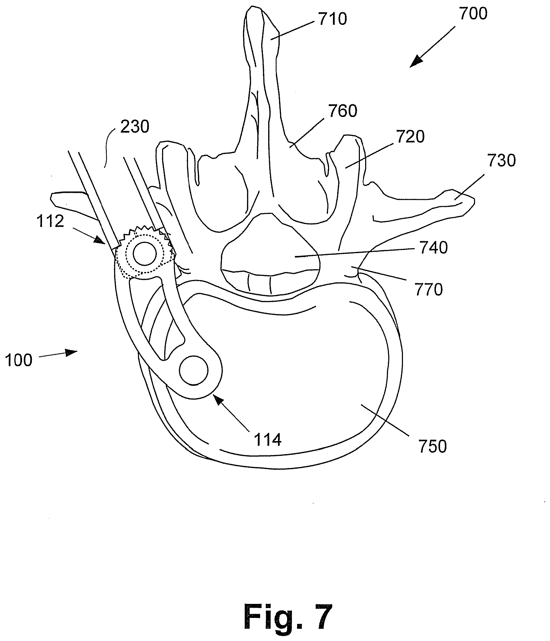

An exemplary method of inserting an interbody spacer using an insertion tool is described in FIG. 6 with reference to interaction between the interbody spacer as described in FIGS. 3A through 5B and the surgical process described in FIGS. 7 through 10. As illustrated in FIG. 6, the present exemplary method begins by the insertion instrument engaging the interbody spacer (step 600) as shown in FIGS. 3A and 3B. To initially engage the interbody spacer, the lever (220, FIG. 3A) is moved to the first position as shown in FIG. 3A. This opens the opposing jaws (240, 250; FIG. 3B). The jaws (240, 250; FIG. 3B) are then inserted into the access gap (122, FIG. 2B) and around the outer perimeter of the transverse cylinder (116, FIG. 3B).

The spacer is then locked at the desired angle (step 610). Once the insertion instrument (200, FIG. 3A) is engaged with the interbody spacer (100, FIG. 3A) in the desired orientation, the lever (220, FIG. 3A) is moved to the third position as shown in FIGS. 5A - 5B. This closes and retracts the jaws (240, 250; FIG. 5B), which pulls the spacer teeth (120, FIG. 5B) into contact with the shaft teeth (260), thereby restraining the interbody spacer (100) from pivoting with respect to the insertion instrument (200).

The insertion instrument with the attached interbody spacer (100) is then inserted into the surgical site (step 620). Now referring to FIG. 7, which illustrates the insertion of the interbody spacer (100) into the intervertebral space. The vertebra (700) comprises a vertebral body (750); a transverse process (730) connected to the vertebral body by a pedicle (770); a superior articular facet (720) connected to the spinous process (710) by a lamina (760). The spinal canal (740) passes between the pedicles (770) anterior to the lamina (760) and spinous process (710). A plurality of nerve foramen (not shown) provide space for nerves to exit the spinal column.

In one exemplary embodiment, the interbody spacer is placed in the anterior space between adjoining vertebral bodies by the TLIF process. However, the present system and method may be used for any number of implant applications. As mentioned above, the TLIF process uses a posterior and lateral approach to access the disc space. To gain posterior and lateral access to the disc space, typically a facet joint is removed. The facet joint consists of a superior articular facet (720) and the inferior articular facet (not shown) of the adjoining vertebra. The required amount of bone removal is determined by the surgeon and varies from patient to patient. The removal of the facet joint allows access via the nerve foramen to the intervertebral space between the two vertebral bodies. After preparatory surgical procedures are performed, the interbody spacer (100) is inserted using the insertion instrument (200).

The spacer (100) enters the surgical site with the distal end (114) leading. As mentioned above, the distal end (114) has a double elliptical shape, which eases the insertion of the spacer into the surgical site.

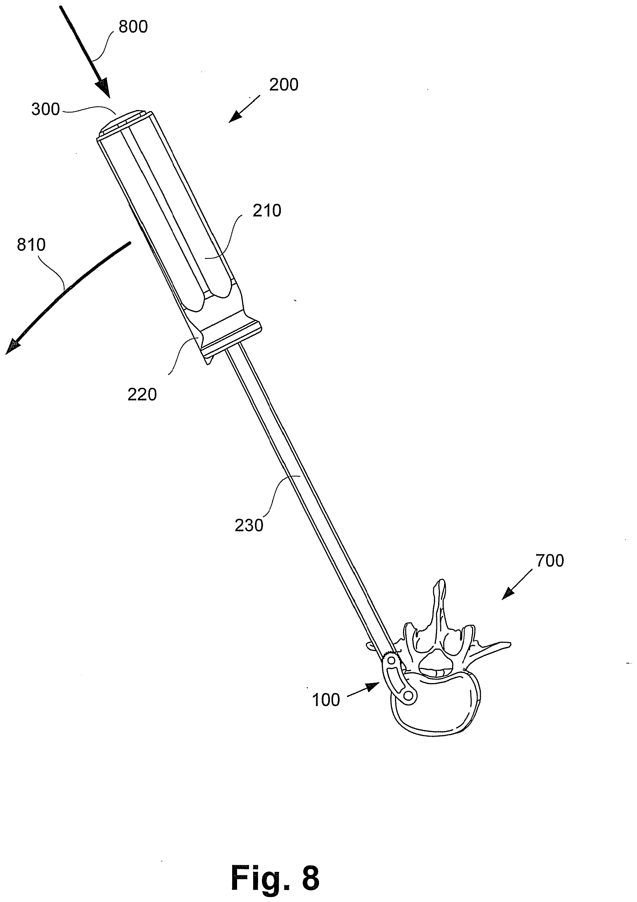

As shown in FIG. 8, the interbody spacer (100) may be inserted using a combination of simultaneous impaction and rotation (step 630, FIG. 6), followed by repositioning of the instrument about the implant. Impaction typically involves striking the impaction cap (300) on the end of handle, either manually or with another instrument as indicated by the straight arrow (800). The curved arrow (810) shows a rigid body rotation of the insertion instrument (200) and the fixably attached interbody spacer (100).

Once the spacer (100) can no longer be inserted in its coupled state, the instrument may be repositioned or pivoted relative to the spacer (100) to allow for further insertion and simultaneous rotation (step 640, FIG. 6). To pivot the insertion instrument (200) about the end of the spacer the lever is moved to the second position, which disengages the matching teeth (120, 260; FIG. 4) as shown in FIG. 4.

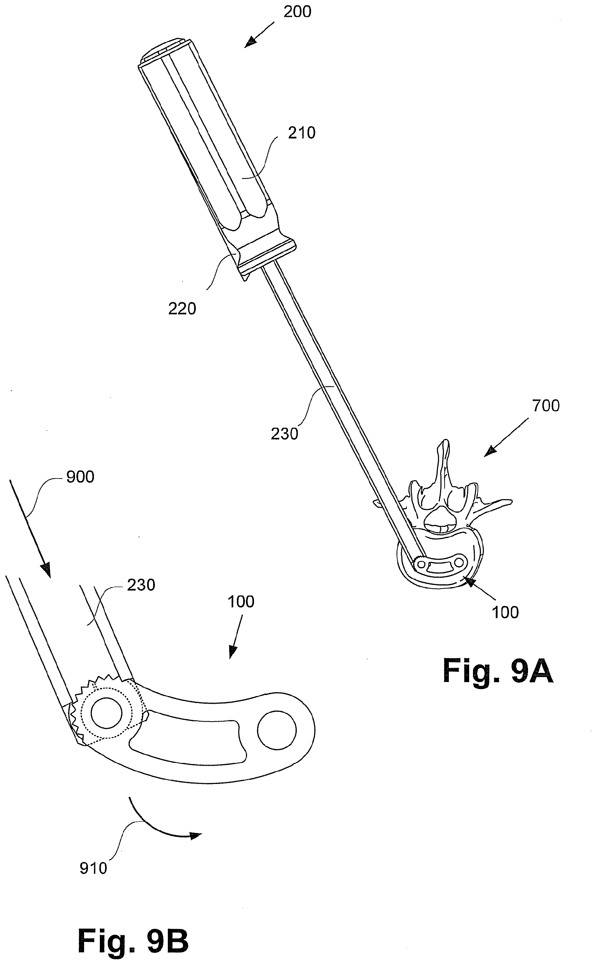

The insertion instrument can then be pivoted with respect to the partially inserted spacer (100) as shown in FIG. 9. The lever (220) is then returned to the third position. FIG. 9B shows the interbody spacer locked into a new angular position after the pivoting process. By pivoting the spacer (100), obstacles can be avoided and narrow or non-linear passageways can be followed without tamping the spacer into place. If required, the spacer can then be further inserted by impacting the insertion instrument as shown by the straight arrow (900) and by further rotation as shown by the curved arrow (910). The steps of pivoting and inserting the spacer are continued until the spacer is in its final position (step 650: FIG. 6).

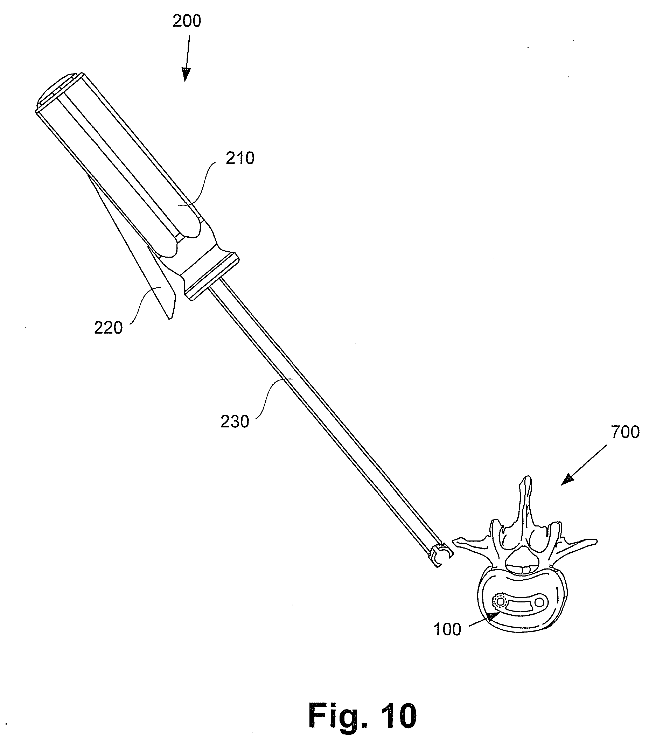

FIG. 10 illustrates the releasing of the spacer and the removal of the insertion instrument (step 660: FIG. 6). Once in a final position, the interbody spacer (100) is released from the insertion instrument (200) by moving the lever to the first position as illustrated in FIG. 10. The jaws (240, 250) open and can then be disengaged from around the outer perimeter of the transverse cylinder (116, FIG. 1). The insertion instrument (200) is removed from the patient, leaving the interbody spacer correctly positioned within the surgical site.

In conclusion, the present exemplary systems and methods provide for a pivotable interbody spacer that provides a user with the ability to insert the spacer in a non-linear path. The insertion instrument can lock onto the interbody spacer at multiple angles to allow for the spacer to be pivoted in increments if the instrument rotation is restricted such that the instrument can only be rotated less than the total rotation required to position the spacer. This additional surgical flexibility can allow insertion of the interbody spacer with the removal of less tissue and bone which results in less invasive surgery, fewer post operative complications, and quicker patient recovery time.

Additionally, the present exemplary interbody spacer (100) employs geometry that provides for a small spacer with relatively large surface area and internal cavity. The spacer is hollowed out to increase cavity volume and surface area while minimizing overall size. Additionally the present exemplary systems and methods allow for rotation of the spacer for final positioning without having to release the spacer and tamp the spacer into place. Because the insertion instrument is not required to be disengaged from the spacer, quicker and simpler surgeries are possible.

The preceding description has been presented only to illustrate and describe the present methods and systems. It is not intended to be exhaustive or to limit the present system and method to any precise form disclosed. Many modifications and variations are possible in light of the above teaching.

The foregoing embodiments were chosen and described in order to illustrate principles of the system and method as well as some practical applications. The preceding description enables others skilled in the art to utilize the method and system in various embodiments and with various modifications as are suited to the particular use contemplated. It is intended that the scope of the present exemplary system and method be defined by the following claims.

## Claims

1. An interbody spacer system comprising an interbody spacer (100), and an insertion instrument (200):
said insertion instrument comprising
a handle member (210);
an extension member (230) including a first and a second end,
wherein said first end of said extension member (230) is coupled to said handle member (210);
a means for selectively coupling a spacer (100) to said second end of said extension member (230);
a means for selectively fixing said spacer (100) in an angular position with respect to said insertion instrument (200);
a means for actuating said means for selectively coupling and said means for selectively fixing a shaft;
said interbody spacer (100) comprising a frictionally engaging feature (120) on at least one end,
said shaft (230) terminating in opposing jaws (240, 250) and frictional features designed to engage with the frictional features on the interbody spacer (100);
wherein the insertion instrument (200) comprises a lever (220) which is mechanically connected to the opposing jaws (240, 250) at the terminal end of the shaft (230) and which is configured for a first position providing an open jaw configuration, a second position providing a closed jaw configuration which allows the spacer to rotate, and a third position providing restrain of the spacer by locking its rotation.

2. The interbody spacer system of claim 1 wherein said lever (220) is configured to selectively extend and retract said pair of protruding jaws (240,250) to grasp and release said interbody spacer (100).

3. The spinal spacer system of claim 2, wherein said lever (220) is configured such that in said first actuation position of said lever said pair of protruding jaws (240,250) extends to a release position and in said second actuation position said pair of protruding jaws (240,250) is disposed in a retracted position locking said interbody spacer in position with respect to said handle member (210).

4. The spinal spacer system of claim 1, wherein: said frictional features of said shaft comprise a first plurality of teeth (260); and said frictional engaging feature of said interbody spacer (120) comprises a second plurality of teeth (120).

## Patentansprüche

1. Zwischenkörper-Abstandselementsystem, umfassend ein Zwischenkörper-Abstandselement (100) und ein Einsetzinstrument (200),
wobei das Einsetzinstrument folgendes umfasst:
ein Griffelement (210);
ein Verlängerungselement (230), einschließlich eines ersten und zweiten Endes, wobei das erste Ende des Verlängerungselements (230) mit dem Griffelement (210) verbunden ist;
ein Mittel zur selektiven Kupplung eines Abstandselements (100) an das zweite Ende des Verlängerungselements (230);
ein Mittel zur selektiven Fixierung des Abstandselements (100) in einer Winkelstellung zu dem Einsetzinstrument (200);
ein Mittel zur Betätigung des Mittels zur selektiven Kupplung und des Mittels zur selektiven Fixierung;
einen Schaft;
wobei das Abstandselement (100) ein Reibeingriffselement (120) an mindestens einem Ende umfasst,
wobei der Schaft (230) in gegenüberliegenden Klemmbacken (240, 250) und Reibungselementen, die dazu ausgelegt sind, in die Reibungselemente an dem Abstandselement (100) einzugreifen, endet;
wobei das Einsetzinstrument (200) einen Hebel (220) umfasst, der mechanisch mit den gegenüberliegenden Klemmbacken (240, 250) am terminalen Ende des Schafts (230) verbunden ist, und der für eine ersten Position, die eine offene Klemmbackenkonfiguration bereitstellt, eine zweite Position, die eine geschlossene Klemmbackenkonfiguration, in welcher das Abstandselement rotieren kann, bereitstellt und eine dritte Position, die das Abstandselement einschränkt, indem sie seine Rotation hindert, ausgelegt ist.

2. Zwischenkörper-Abstandselementsystem nach Anspruch 1, wobei der Hebel (220) dazu ausgelegt ist, das hervorstehende Klemmbackenpaar (240, 250) selektiv auszufahren und einzuziehen, um das Zwischenkörper-Abstandselement (100) zu greifen und freizugeben.

3. Zwischenkörper-Abstandselement nach Anspruch 2, wobei der Hebel (220) dazu ausgelegt ist, dass das hervorstehende Klemmbackenpaar (240, 250) in der ersten Betätigungsposition des Hebels zu einer Freigebungsposition ausgefahren wird, und das hervorstehende Klemmbackenpaar (240, 250) in der zweiten Betätigungsposition in einer eingezogenen Position angeordnet ist, wobei das Zwischenkörper-Abstandselement in seiner Position in Bezug auf das Griffelement (210) fixiert ist.

4. Zwischenkörper-Abstandselementsystem nach Anspruch 1, wobei:
die Reibungselemente des Schafts eine erste Vielzahl an Zähnen (260) umfassen; und wobei das Reibeingriffselement des Zwischenkörper-Abstandselements (120) eine zweite Vielzahl an Zähnen (120) umfasst.

## Revendications

1. Système d'espaceur vertébral comprenant un espaceur vertébral (100) et un instrument d'insertion (200) :
ledit instrument d'insertion comprenant un membre de poignée (210) ;
un membre d'extension (230) incluant une première et une seconde extrémité,
dans lequel ladite première extrémité dudit membre d'extension (230) est couplée audit membre de poignée (210) ;
un moyen pour coupler de manière sélective un écarteur (100) à ladite seconde extrémité dudit membre d'extension (230) ;
un moyen pour fixer de manière sélective ledit écarteur (100) dans une position angulaire par rapport audit instrument d'insertion (200) ;
un moyen pour déclencher ledit moyen pour coupler de manière sélective et ledit moyen pour fixer de manière sélective ;
une tige ;
ledit espaceur vertébral (100) comprenant une fonctionnalité d'engagement par friction (120) sur au moins une extrémité,
ladite tige (230) terminant en des mâchoires opposées (240, 250) et les fonctionnalités par friction étant conçues pour s'engager avec les fonctionnalités par friction sur l'espaceur vertébral (100) ;
dans lequel l'instrument d'insertion (200) comprend un levier (220) qui est relié mécaniquement aux mâchoires opposées (240, 250) sur l'extrémité de fin de la tige (230) et qui est configuré pour une première position fournissant une configuration de mâchoires ouvertes, une deuxième position fournissant une configuration de mâchoires fermées qui permet à l'espaceur de tourner, et une troisième position fournissant une retenue de l'espaceur en verrouillant sa rotation.

2. Espaceur vertébral selon la revendication 1, dans lequel ledit levier (220) est configuré pour étendre et rétracter de manière sélective ladite paire de mâchoires saillantes (240, 250) pour saisir et libérer ledit espaceur vertébral (100).

3. Espaceur vertébral selon la revendication 2, dans lequel ledit levier (220) est configuré de telle façon que dans ladite première position de déclenchement dudit levier, ladite paire de mâchoires saillantes (240, 250) s'étend vers une position de libération et dans ladite deuxième position, ladite paire de mâchoires saillantes (240, 250) est disposée dans une position rétractée verrouillant l'espaceur vertébral en place par rapport audit membre de poignée (210).

4. Espaceur vertébral selon la revendication 1, dans lequel :
lesdites fonctionnalités par friction de ladite tige comprennent
une première pluralité de dents (260) ; et ladite fonctionnalité d'engagement par friction dudit espaceur vertébral (120) comprend une seconde pluralité de dents (120).
